# EUROPEAN PATENT SPECIFICATION

(11) **EP 0 862 410 B1**
(45) Date of publication and mention of the grant of the patent: **18.12.2002**
(21) Application number: 96934606.3
(22) Date of filing: 09.10.1996
(51) Int. Cl.: A61K 7/48

(54) **COSMETIC COMPOSITIONS**
KOSMETISCHE ZUSAMMENSETZUNGEN
COMPOSITIONS COSMETIQUES

(30) Priority: 10.10.1995 GB 9520690
(43) Date of publication of application: 09.09.1998
(73) Proprietor: The Boots Company PLC, Nottingham, Nottinghamshire NG2 3AA (GB)
(72) Inventor: SHEARD, Christine, Nottingham NG2 3AA (GB)
(74) Representative: Jones, Stephen Anthony
(86) International application number: EP9604393
(87) International publication number: WO97013497

(56) References cited:
- EP-A- 0 502 769
- WO-A-94/15580
- WO-A-94/28860
- WO-A-96/17592
- US-A- 5 534 247

## Description

This invention relates to novel cosmetic compositions for topical application, in particular to novel cosmetic compositions for application to the lips or skin.

A cosmetic composition (for example a concealer, blusher, eye shadow, mascara, foundation, lipstick or other make-up) should possess certain physical properties to be acceptable to a consumer. It should, for example have a suitable texture to permit smooth and rapid application with the minimum of pressure. The applied cosmetic film should be resistant to wear encountered during use (for example a lipstick should resist mild abrasion during eating, drinking, or smoking). For a lipstick composition it is important that the ingredients used have acceptable organoleptic properties (i.e. are not bitter or astringent tasting). If the cosmetic composition is pigmented (i.e. used to tint or colour the body) it is particularly important that the composition should be sufficiently substantive such that colour is retained only on that portion of the body to which it is applied, and does not spread colour onto the adjacent skin (for example a lipstick should not spread away from the lips).

Use of ingredients having improved substantive properties to formulate cosmetic compositions is desirable for several reasons. The interval between re-application of cosmetic colour may be increased and the rub off of coloured cosmetic to other surfaces (for example clothing which may require cleaning) may be reduced. The resilience of the cosmetic film formed on the skin or lips may be improved. A more resilient film may also reduce water loss from the skin which may produce an enhanced moisturising and/or conditioning effect.

Thus it can be seen that cosmetic compositions comprising ingredients with improved substantive properties may provide some or all of the aforementioned advantages.

WO-A-9415580 discloses oil-in-water emulsions containing surface-treated pigments, a carboxylic acid polymer thickener, a soluble chemical sunscreen agent and a humectant to provide colour, moisturisation and protection from UV radiation.

WO-A-94228860 discloses make-up compositions comprising water-in-oil emulsions containing a silicone phase, humectant, pigment and organic amphiphilic material capable of forming smectic lyotropic liquid crystals in the product or on the skin.

EP-A-502769 discloses cosmetic compositions comprising a dispersion in a fatty binder of a particulate filler, 50% of which comprises spherical particles between 0.5 and 50 microns in size. The composition is said to blur skin defects whilst leaving a translucent layer on the skin.

Lipstick compositions are known which comprise oil compatible, cationic, quaternary compounds (for example those available under the trade name Incroquat Behenyl TMS [from Croda Surfactants Ltd]). Such cationic ingredients do not form a satisfactory film when applied to the lips. Other lipstick compositions are known which use as the film-forming ingredient non-ionic, oil soluble ingredients such as the PVP copolymer available commercially under the trade name Antaron V220 [from GAF (Great Britain) Co. Ltd]. These known lipstick compositions do not teach the novel cosmetic compositions of the present invention or suggest the advantages of using hydrophilic cationic resins as a substantive ingredient in such cosmetic compositions.

It had previously been thought that hydrophilic cationic resins could not be used as a substantive ingredient in hydrophobic cosmetic compositions (such as lipsticks) as it was believed that they could not be incorporated into an oily base to form a stable composition. Surprisingly it has now been found that hydrophilic cationic resins are suitable for use in such cosmetic compositions as a substantive ingredient. Such cationic resins have been found to be particularly effective at adhering to the lips or skin, and are therefore particularly effective as the substantive film-forming ingredient in lipsticks, to improve the lipsticks' conditioning ability, moisturising ability and durability. These cationic resins may also act on the other ingredients in a hydrophobic cosmetic formulation to synergistically enhance topical delivery of the composition and/or the active ingredients therein (e.g. pigment).

Therefore, broadly in accordance with the present invention there is provided a cosmetic composition comprising:
from 0.05% to 5% w/w of a hydrophilic cationic resin;
from 30% to 85% w/w of an oil component;
from 1 % to 40% w/w of a wax component; and
from 1 % to 40% w/w of a powder component.

Preferably the cosmetic composition comprises a lipstick composition.

The term 'w/w' as used herein indicates the percentage mass of ingredient per total mass of the composition.

Preferably the hydrophilic cationic resin may be water soluble and/or water swellable.

More preferably the hydrophilic cationic resin comprises one or more polyquaternium polymer or polymers. Polyquaternium polymers are denoted by the Cosmetic Toiletries and Fragrance Association (CTFA) name polyquaternium followed by a number. Generally polyquaternium polymers comprise quaternary ammonium polymers, polymeric quaternary ammonium salts or are formed from such polymers or polymeric salts. It will be understood therefore that the hydrophilic cationic resin may be any suitable polymeric quaternary ammonium polymer, polymeric quaternary ammonium salt or any mixtures thereof. The hydrophilic cationic resin may be a homopolymer formed from the same monomer type or may be a co-polymer formed from two or more different monomer types.

Examples of polymers or polymeric salts suitable for use as the hydrophilic cationic resin in the present invention comprise one or more of the following and/or any suitable mixtures thereof: polyquaternium-1 (a polymeric quaternary ammonium salt); polyquaternium-2 (a polymeric quaternary ammonium salt); polyquaternium-4 (a copolymer of hydroxyethylcellulose and diallyldimethyl ammonium chloride); polyquaternium-5 (the co-polymer of acrylamide and β-methacrylyloxyethyl trimethyl ammonium methosulphate); polyquaternium-6 (a polymer of dimethyl diallyl ammonium chloride); polyquaternium-7 (the polymeric quaternary ammonium salt consisting of acrylamide and dimethyl diallyl ammonium chloride monomers); polyquaternium-8 (the polymeric quaternary ammonium salt of methyl and stearyl dimethylaminoethyl methacrylate quaternised with dimethyl sulphate); polyquaternium-9 (the polymeric quaternary ammonium salt of polydimethylaminoethyl methacrylate quaternised with methyl bromide); polyquaternium-10 (a polymeric quaternary ammonium salt of hydroxyethylcellulose reacted with a trimethyl ammonium substituted epoxide); polyquaternium-11 (a quaternary ammonium polymer formed by the reaction of diethyl sulphate and a copolymer of vinylpyrrolidine and dimethyl aminoethylmethacrylate); polyquaternium-12 (a polymeric quaternary ammonium salt prepared by the reaction of ethyl methacrylate / abletyl methacrylate / diethylaminoethyl methacrylate copolymer with dimethyl sulphate); polyquaternium-13 (a polymeric quaternary ammonium salt prepared by the reaction of ethyl methacrylate / oleyl methacrylate / diethylaminoethyl methacrylate copolymer with dimethyl sulphate); polyquaternium-14 (a polymeric quaternary ammonium salt); polyquaternium-15 (the copolymer of acryloxide and β-methacrylyloxyethyl trimethyl ammonium chloride); polyquaternium-16 (a polymeric quaternary salt formed from methylvinylimidazolium chloride and vinylpyrrolidine); polyquaternium-17 (a polymeric quaternary salt prepared by the reaction of adipic acid and dimethylaminopropylamine reacted with dichloroethyl ether); polyquaternium-18 (a polymeric quaternary salt prepared by the reaction of azelaic acid and dimethylaminopropylamine reacted with dichloroethyl ether); polyquaternium-19 (a polymeric quaternary ammonium salt prepared by the reaction of polyvinyl alcohol with 2,3-epoxypropylamine); polyquaternium-20 (a polymeric quaternary ammonium salt prepared by the reaction of polyvinyl octadecyl ether with 2,3-epoxypropylamine); polyquaterium-22 (a copolymer of dimethyldiallyl ammonium chloride and acrylic acid); polyquaternium-24 (a polymeric quaternary ammonium salt of hydroxyethylcellulose reacted with a lauryl dimethyl ammonium substituted epoxide); polyquaternium-27 (the block copolymer formed by reacting polyquaternium-2 [q.v.] with polyquaternium-17 [q.v.]); polyquaternium-28 (a polymeric quaternary ammonium salt consisting of vinylpyrrolidone and dimethylaminopropyl methacrylamide monomers); polyquaternium-29 (Chltosan that has been reacted with propylene oxide and quaternised with epichlorohydrin); polyquaternium-30 (a polymeric quaternary ammonium salt); polyquaternium-31 (a polymeric quaternary salt prepared by the reaction of DMAPA acrylates / acryclic acid / acrylonitrogens copolymer and diethyl sulphate); polyquaternium-32 (a polymeric quaternary ammonium salt); polyquaternium-33 (a polymeric quaternary ammonium salt); polyquaternium-34 (a polymeric quaternary ammonium salt); polyquaternium-35 (a polymeric quaternary ammonium salt); polyquaternium-36 (a polymeric quaternary ammonium salt); polyquaternium-37 (a polymeric quaternary ammonium salt); and polyquaternium-39 (a polymeric quaternary ammonium salt of acrylic acid, diallyl dimethyl ammonium chloride and acrylamide).

Most preferably the hydrophilic cationic resin is polyquaternium-37, such as that available commercially from Allied Colloid under the trade names Salcare SC95 or Salcare SC96.

The hydrophilic cationic resin may comprise a suitable base material for example selected from an emollient ester and/or an emollient oil.

The oil component may comprise one or more cosmetically acceptable oils, silicones, fats and any mixtures thereof.

Suitable cosmetically acceptable oils comprise natural oils (for example plant oils [such as almond oil, vegetable oil and castor oil]), synthetic oils (for example octyl dodecanol and/or fatty esters [such as pentaerythrityl tetracaprylate and pentaerythrityl tetraisostearate]), fatty oils (for example triglyceride esters), fatty alcohols (for example oleyl alcohol and octyldodecanol) and mineral oils (for example liquid paraffin and C₁₁₋₁₂ isoparaffin). Suitable cosmetically acceptable silicones may comprise volatile silicones (for example volatile linear polydimethylsiloxanes) and cyclomethicone and non-volatile silicone oils (for example phenyldimethicone).

Suitable cosmetically acceptable fats may comprise natural fats (for example animal fats [such as wool fat], vegetable fats [such as triglyceride esters] and mineral fats [such as white soft paraffin]) and synthetic fats (for example bisdiglyceryl caprylate / caprate / isostearate / stearate / hydroxystearate adipate).

Preferably the oil component is present in an amount from 50% to 80% w/w.

Optionally the oil component may further comprise soothing agents (for example alpha bisabalol and/or a plant extract [such as chamomile extract and/or liquorice extract]) and/or moisturising agents (for example an alkylene glycol [such as butylene glycol]).

The wax component has a melting point in the range from about 30 °C to about 120°C. Suitably the wax component comprises one or more cosmetically acceptable waxes, wax-like materials and any mixtures thereof. Suitable waxes comprise natural waxes (for example plant waxes [such as candelilla wax, carnauba wax and illupe butter], animal waxes [such as beeswax, lanolin wax and lanolin derivative waxes] and mineral waxes [such as ozokerite wax, montan wax and paraffin waxes {e.g. microcrystalline wax and paraffin}]), synthetic waxes (for example hydrogenated castor oil, hydrocarbon wax and cetyl alcohol) and silicone waxes (for example C₂₄₋₂₈ alkyl methicone).

Preferably the wax component is present in the cosmetic composition in an amount from 5% to 40% w/w, more preferably from 10% to 30% w/w.

The powder component of the invention is generally a dry, particulate matter having a mean primary particle size from 0.02 microns to 80 microns. The particle size is measured along the longest axis of the particle.

The particulate matter may be pigmentary powder and/or non-pigmentary powder.

Preferably, the powder component is present in an amount from 2% to 30% w/w. This is particularly advantageous if the cosmetic composition is a lipstick.

Suitable non-pigmentary powders comprise acrylate polymers, alumina, aluminium silicate, bentonite, boron nitride, calcium carbonate, calcium silicate, cellulose, corn starch, kaolin, magnesium aluminium silicate, magnesium carbonate, mica, micronised Teflon® (PTFE), nut shell powder (such as walnut shell powder), nylon, polyethylene, polymethyl methacrylate beads, silica, silk powder, tin oxide, zinc myristate and any mixtures thereof.

Suitable pigmentary powders comprise various inorganic and organic pigments. Suitable inorganic pigments, especially for use in lipsticks, comprise iron oxides, ultramarines and titanium dioxide. The powder component may further comprise inorganic pigments that give a pearly finish, for example bismuth oxychloride and titanium dioxide coated mica. Suitable organic pigments, especially for use in lipsticks, comprise various suitable aromatic components including monoazo, indigoid, fluoran and pyrazole dyes. The pigmentary powders are selected to be cosmetically acceptable and to comply with the laws and regulations of the territories in which they are to be used. For example in the European Union at the priority date of the present application, suitable pigments are those listed in Annex IV of the Cosmetics Directive 76/768 EEC.

The percentage of pigmentary powder used in the powder component will depend upon the type of cosmetic being formulated. Blushers, eyeshadows, mascara, lipsticks and similar cosmetics will contain higher percentages of pigment in the powder component, usually above 5% w/w of the total cosmetic composition.

The above-mentioned powders (either pigmentary or non-pigmentary) may be surface-treated with aluminium stearate, amino acids, mineral oils, phospholipids (for example lecithin) silicone oil or various other agents, either alone or in combination, which coat the powder surface, render the particles hydrophobic in nature, prevent agglomeration and/or aid dispersion of the powder.

The cosmetic composition of the present invention may also comprise, in additional to the pigment an additional dye to achieve the desired shade and/or colour in the resultant product (e.g. suitable and acceptable dyestuffs such as those known by the names Red 21, Red 27 and/or Orange 27).

Optionally the cosmetic composition further comprises one or more preservatives, antioxidants, and/or perfumes. Any conventional preservative, anti-oxidant and perfume may be employed. Optionally such a scavenger of free radicals may be added which may also be an anti-oxidant and/or preservative and/or have additional desirable properties (for example a vitamin, such as vitamin E). Preferred preservatives and/or antioxidants are selected from one or more of parabens, butylated hydroxy toluene, butylated hydroxyannisole and propyl-p-hydroxybenzoate.

The cosmetic composition of the present invention is preferably a lipstick and may further comprise one or more cosmetically acceptable additional ingredients suitable for lipsticks selected from: gloss modifiers, texture modifiers, pearlising agents (e.g. titanium dioxide coated mica [also known as timica]), matting agents (e.g. nylon powder), moisturising agents, soothing agents, conditioners, vitamins, sunscreens and/or suitable mixtures thereof.

The term "lipsticks" as used herein includes medicated and unmedicated lip salves, lip balms, lip glosses as well as lipsticks to colour the lips.

The term "sunscreen" is used herein to encompass tanning lotions, sunscreens and sunblockers which are intended for use on the body to provide protection against the sun's rays or other UV sources. Sunscreen agents which may be incorporated into the powders and / or compositions of the present invention comprise organic chemical agents which act to absorb incident UV radiation and / or may be inorganic sunscreen agents which act to reflect incident UV radiation. Examples of suitable sunscreening agents comprise one or more of the following or any suitable mixtures thereof: p-aminobenzoic acids, esters and derivatives (e.g. 2-ethylhexyl p-dimethylaminobenzoate or the octyl ester of p-aminobenzoic acid); methoxycinnamate esters (e.g. 2-ethylhexyl- p-methoxycinnamate, or 2-ethoxyethyl-p-methoxycinnamate); benzophenones (for example oxybenzone); dibenzoylmethanes (e.g. butylmethoxy dibenzoyl methane); salicylate esters; TiO₂ (preferably of a particle size of from 1 to 100 nm); and ZnO (preferably of a particle size of from 1 to 50 nm). Preferably any sunscreening agent is present in an amount from 0.1% to 25%, more preferably 0.5% to 10% by weight of the composition.

Preferably the cosmetic composition of the present invention is solid at ambient temperature.

Broadly in accordance with a further aspect of the present invention, there is provided a cosmetic composition as described herein in association with a suitable receptacle or dispenser thereof.

Broadly, in a still further aspect of the present invention broadly there is provided a method for cosmetic treatment comprising topical administration of a cosmetic composition as described herein.

In a yet further aspect of the present invention there is provided a process for preparing a cosmetic composition as described herein, the process comprising mixing intimately together the following ingredients: from 0.05% to 5% w/w of a hydrophilic cationic resin;
from 30% to 85% w/w of an oil component;
from 1% to 40% w/w of a wax component; and
from 1% to 40% w/w of a powder component.

Preferably the process of the present invention comprises a further step of moulding the mixture into a solid form suitable for topical application.

The present invention will now be illustrated by the following non-limiting examples. With the exception of the cationic resin, the other ingredients used in these examples are conventional lipstick ingredients which may be substituted by other similar ingredients (for example those described herein) as is well known to those skilled in the art. Thus these conventional ingredients need not be specified further. Due to rounding the weight percentages given in the examples may not total exactly 100 %.

### Example 1

A lipstick was prepared to the following composition:

| *Ingredient* | *% w*/*w* |
|---|---|
| Plant wax | 6.4 |
| Paraffin wax | 9.0 |
| Synthetic wax | 2.3 |
| Synthetic fat | 10.0 |
| Fatty alcohol | 20.7 |
| Synthetic ester | 12.74 |
| Plant oil | 26.24 |
| Preservative | 0.1 |
| Antioxidant | 0.03 |
| Cationic water soluble resin (available from Allied Colloids under | |
| the tradename 'Salcare SC96') | 2.25 |
| Butylene glycol | 1.5 |
| Pigment | 8.74 |

The lipstick was prepared as follows. The pigment was premilled into the plant oil ingredient. The waxes and synthetic fat were melted together. The alcohol and ester were added to the melted wax and the mixture was stirred until uniform. The preservative and antioxidant were added to the mixture and dissolved. The plant oil (comprising the pigment) was added to the wax mixture with high sheer mixing followed by the cationic resin, which was added with continued high sheer mixing. The resultant composition was poured into a mould to form a lipstick.

The above lipstick was compared to a bare skin control in the following test.

To the volar aspect of the forearm of eleven volunteers, two sites were delineated, one to which the lipstick was applied and the other which was left untreated as a control. Six corneometer measurements were taken from both sites for each time interval. The lipstick was applied at a rate of about 2 mg cm⁻². After time intervals of two hours, the lipstick was removed by gently wiping the site of application with a cotton bud. The control site was wiped in the same way. Ten minutes later corneometer measurements were taken from each site. The results are tabulated below.

| Composition | % change in hydration of the skin after: | | | |
|---|---|---|---|---|
| | 2 hours | 4 hours | 6 hours | 8 hours |
| Example 1 | 8.69% | 7.02% | 5.57% | 5.80% |
| Untreated Skin | 1.70% | 0.18% | 2.62% | 1.21% |

Thus the net percentage increase in skin hydration for the site treated with lipstick compared to untreated skin is given below:

| Net percentage increase after: | | | | |
|---|---|---|---|---|
| | 2 hours | 4 hours | 6 hours | 8 hours |
| Example 1 | 7.49% | 6.76% | 3.48% | 4.39% |

The results were analysed using the one way analysis of variance followed by Krushal-Willis and the Millers Comparison test. The results point to an increase in skin moisture of the lipstick treated skin, compared to untreated skin, of up to 7% after 1 hour and up to 4% after 8 hours. This is due to the substantive qualities of the lipstick which forms a substantive, resilient film on the skin, which reduces the amount of water evaporated from the skin. Thus these results show the lipstick of Example 1 acts to moisturise and condition the skin.

### Example 2

A pearl shade lipstick was prepared to the following composition:

| *Ingredient* | *% w*/*w* |
|---|---|
| Plant wax | 4.2 |
| Mineral wax | 5.7 |
| Synthetic wax | 8.7 |
| Plant oil | 20.2 |
| Synthetic oil | 36.9 |
| Organic sunscreens | 2.25 |
| Soothing agents | 1.01 |
| Antioxidant | 0.03 |
| Tocopheryl acetate | 0.5 |
| Pigment | 7.75 |
| Dye | 0.25 |
| Timica | 12.0 |
| Cationic water soluble resin (available from Allied Colloids under the tradename 'Salcare SC96') | 0.5 |

The lipstick was prepared as follows. The pigment and dye were premilled into the plant oil ingredient. The waxes and other solid fatty materials were heated together and mixed until uniform. If the synthetic oil comprises a fatty ester, the ester is added to the melted wax and the mixture was stirred until uniform. The antioxidant was added to the mixture and dissolved. The cationic resin and soothing agent were added to the wax mixture with high sheer mixing the pigment, dye and timica were dispersed in the remaining synthetic oil and added to the bulk mixture using high shear mixing to achieve the desired shade and effect. The sunscreens were added followed by the tocopheryl acetate. The resultant composition was poured into a mould to form a lipstick which had similar advantageous properties to the lipstick described in Example 1.

### Example 3

A plain shade lipstick was prepared to the following composition:

| *Ingredient* | *% w*/*w* |
|---|---|
| Plant wax | 4.4 |
| Mineral wax | 6.2 |
| Synthetic wax | 9.5 |
| Plant oil | 22.0 |
| Synthetic oil | 44.2 |
| Organic sunscreens | 2.4 |
| Soothing agents | 1.01 |
| Antioxidant | 0.03 |
| Tocopheryl acetate | 0.5 |
| Pigment | 9.0 |
| Dye | 0.25 |
| Cationic water soluble resin (available from Allied Colloids under the tradename 'Salcare SC96') | 0.5 |

This lipstick was prepared in an analogous manner to the lipstick described in Example 2 and had similar advantageous properties to the lipstick described in Example 1.

### Example 4

A matt shade lipstick was prepared to the following composition:

| *Ingredient* | *% w*/*w* |
|---|---|
| Plant wax | 4.0 |
| Mineral wax | 5.4 |
| Synthetic wax | 8.2 |
| Plant oil | 19.0 |
| Synthetic oil | 38.6 |
| Organic sunscreens | 2.1 |
| Soothing agents | 1.01 |
| Antioxidant | 0.03 |
| Tocopheryl acetate | 0.5 |
| Pigment | 5.75 |
| Dye | 0.16 |
| Timica | 2.8 |
| Nylon powder (matting agent) | 12 |
| Cationic water soluble resin (available from Allied Colloids under the tradename 'Salcare SC96') | 0.5 |

This lipstick was prepared in an analogous manner to the lipstick described in Example 2 and had similar advantageous properties to the lipstick described in Example 1.

## Claims

1. A cosmetic composition comprising
from 0.05 % to 5 % w/w of a hydrophilic cationic resin;
from 30 % to 85 % w/w of an oil component;
from 1 % to 40 % w/w of a wax component; and
from 1 % to 40 % w/w of a powder component.

2. A cosmetic composition as claimed in claim 1, in which the hydrophilic cationic resin is water soluble and/or water swellable.

3. A cosmetic composition as claimed in any preceding claim, in which the hydrophilic cationic resin comprises one or more polyquaternium polymers and/or polymeric salts.

4. A cosmetic composition as claimed in any preceding claim, in which the hydrophilic cationic resin comprises any suitable polymeric quaternary ammonium polymer, polymeric quaternary ammonium salt or any mixtures thereof.

5. A cosmetic composition as claimed in any preceding claim, in which the hydrophilic cationic resin comprises a homopolymer, a copolymer or any suitable mixtures thereof.

6. A cosmetic composition as claimed in any preceding claim, in which the hydrophilic cationic resin comprises one or more the following or any mixtures thereof:
polyquaternium-1; polyquaternium-2; polyquaternium-4; polyquaternium-5; polyquaternium-6; polyquaternium-7; polyquaternium-8; polyquaternium-9; polyquaternium-10; polyquaternium-11; polyquaternium-12; polyquaternium-13; polyquaternium-14; polyquaternium-15; polyquaternium-16; polyquaternium-17; polyquaternium-18; polyquaternium-19; polyquaternium-20; polyquaternium-22; polyquaternium-24; polyquaternium-27; polyquaternium-28; polyquaternium-29; polyquaternium-30; polyquaternium-31; polyquaternium-32; polyquaternium-33; polyquaternium-34; polyquaternium-35; polyquaternium-36; polyquaternium-37; and
polyquaternium-39.

7. A cosmetic composition as claimed in any preceding claim in which the hydrophilic cationic resin comprises polyquaternium-37.

8. A cosmetic composition as claimed in any preceding claim in which the hydrophilic cationic resin comprises a base material selected from an emollient ester and/or an emollient oil.

9. A cosmetic composition as claimed in any preceding claim, which is solid at ambient temperature and is suitable for use as a lipstick.

10. A cosmetic composition as claimed in any preceding claim, in which the oil component comprises from 50 % to 80 % w/w.

11. A cosmetic composition as claimed in any preceding claim, in which the wax component comprises from 5 % to 40 % w/w.

12. A cosmetic composition as claimed in any preceding claim, in which the wax component comprises from 10 % to 30 % w/w.

13. A cosmetic composition as claimed in any preceding claim, in which the powder component comprises a non-pigmentary powder in conjunction with a pigmentary powder.

14. A cosmetic composition as claimed in any preceding claim, in which the powder component comprises from 2 % to 30 % w/w.

15. A cosmetic composition as claimed in any preceding claim, in which the powder component is surface treated with one or more ingredients selected from aluminium stearate, amino acids, mineral oils, phospholipids, silicone oils and any mixtures thereof.

16. A cosmetic composition as claimed in any preceding claim, which further comprises one or more ingredient or ingredients selected from: gloss modifiers, texture modifiers, moisturising agents, soothing agents, conditioners, vitamins, sunscreens, preservatives, antioxidants, perfumes and any mixtures thereof.

17. A cosmetic product comprising a cosmetic composition as claimed in any preceding claim, in association with a suitable receptacle or dispenser thereof.

18. A method of cosmetic treatment comprising topical administration of a cosmetic composition as claimed in any of claims 1 to 16 or use of a cosmetic product as claimed in claim 17.

19. A process for preparing a cosmetic composition as claimed in any of claims 1 to 17, comprising mixing intimately together the following ingredients:
from 0.05 % to 5 % w/w of water soluble cationic resin;
from 30 % to 85 % w/w of an oil component;
from 1 % to 40 % w/w of a wax component; and
from 1 % to 40 % w/w of a powder component.

20. A process as claimed in claim 19, comprising the further step of moulding the resultant mixture into a solid form suitable for topical application.

## Patentansprüche

1. Kosmetikzusammensetzung, umfassend:
0,05 bis 5 Gew.-% eines hydrophilen kationischen Harzes;
30 bis 85 Gew.-% einer Ölkomponente;
1 bis 40 Gew.-% einer Wachskomponente; und
1 bis 40 Gew.-% einer Pulverkomponente.

2. Kosmetikzusammensetzung nach Anspruch 1, wobei das hydrophile kationische Harz wasserlöslich und/oder in Wasser quellbar ist.

3. Kosmetikzusammensetzung nach einem der vorherigen Ansprüche, wobei das hydrophile kationische Harz ein oder mehrere Polyquaterniumpolymer(e) und/oder polymere(s) Salz(e) umfasst.

4. Kosmetikzusammensetzung nach einem der vorherigen Ansprüche, wobei das hydrophile kationische Harz ein beliebiges polymeres quartäres Ammoniumpolymer, polymeres quartäres Ammoniumsalz oder ein beliebiges Gemisch davon umfasst.

5. Kosmetikzusammensetzung nach einem der vorherigen Ansprüche, wobei das hydrophile kationische Harz ein Homopolymer, ein Copolymer oder ein beliebiges geeignetes Gemisch davon umfasst.

6. Kosmetikzusammensetzung nach einem der vorherigen Ansprüche, wobei das hydrophile kationische Harz ein oder mehrere der folgenden oder irgendwelche Gemische davon umfasst:
Polyquaternium-1; Polyquaternium-2; Polyquaternium-4; Polyquaternium-5; Polyquaternium-6; Polyquaternium-7; Polyquaternium-8; Polyquaternium-9; Polyquaternium-10; Polyquaternium-11; Polyquaternium-12; Polyquaternium-13; Polyquaternium-14; Polyquaternium-15; Polyquaternium-16; Polyquaternium-17; Polyquaternium-18; Polyquaternium-19; Polyquaternium-20; Polyquaternium-22; Polyquaternium-24; Polyquaternium-27; Polyquaternium-28; Polyquaternium-29; Polyquaternium-30; Polyquaternium-31; Polyquaternium-32; Polyquaternium-33; Polyquaternium-34; Polyquaternium-35; Polyquaternium-36; Polyquaternium-37; und Polyquaternium-39.

7. Kosmetikzusammensetzung nach einem der vorherigen Ansprüche, wobei das hydrophile kationische Harz Polyquaternium-37 umfasst.

8. Kosmetikzusammensetzung nach einem der vorherigen Ansprüche, wobei das hydrophile kationische Harz ein Basismaterial umfasst, ausgewählt aus einem Erweichungsester und/oder einem Erweichungsöl.

9. Kosmetikzusammensetzung nach einem der vorherigen Ansprüche, die bei Umgebungstemperatur fest und zur Verwendung als Lippenstift geeignet ist.

10. Kosmetikzusammensetzung nach einem der vorherigen Ansprüche, wobei die Ölkomponente 50 bis 80 Gew.-% umfasst.

11. Kosmetikzusammensetzung nach einem der vorherigen Ansprüche, wobei die Wachskomponente 5 bis 40 Gew.-% umfasst.

12. Kosmetikzusammensetzung nach einem der vorherigen Ansprüche, wobei die Wachskomponente 10 bis 30 Gew.-% umfasst.

13. Kosmetikzusammensetzung nach einem der vorherigen Ansprüche, wobei die Pulverkomponente ein nichtpigmentiertes Pulver in Verbindung mit einem pigmentierten Pulver umfasst.

14. Kosmetikzusammensetzung nach einem der vorherigen Ansprüche, wobei die Pulverkomponente 2 bis 30 Gew.-% umfasst.

15. Kosmetikzusammensetzung nach einem der vorherigen Ansprüche, wobei die Pulverkomponente mit einem oder mehreren Inhaltsstoffen oberflächenbehandelt wird, ausgewählt aus Aluminiumstearat, Aminosäuren, Mineralölen, Phospholipiden, Silikonölen und beliebigen Gemischen davon.

16. Kosmetikzusammensetzung nach einem der vorherigen Ansprüche, die ferner einen oder mehrere Inhaltsstoff(e) umfasst, ausgewählt aus: Glanzmodifizierer, Texturmodifizierer, feuchtigkeitsspendende Mittel, beruhigende Mittel, Pflegemittel, Vitamine, Sonnenschutzmittel, Konservierungsmittel, Antioxidationsmittel, Parfüme und beliebige Gemische davon.

17. Kosmetikprodukt, umfassend eine Kosmetikzusammensetzung nach einem der vorherigen Ansprüche, in Verbindung mit einem geeigneten Behälter oder Spender dafür.

18. Verfahren zur kosmetischen Behandlung, umfassend das topische Verabreichen einer Kosmetikzusammensetzung nach einem der Ansprüche 1 bis 16 oder die Verwendung eines Kosmetikproduktes nach Anspruch 17.

19. Prozess zur Herstellung einer Kosmetikzusammensetzung nach einem der Ansprüche 1 bis 17, umfassend das sofortige Vermischen der folgenden Inhaltsstoffe miteinander:
0,05 bis 5 Gew.-% eines wasserlöslichen kationischen Harzes;
30 bis 85 Gew.-% einer Ölkomponente;
1 bis 40 Gew.-% einer Wachskomponente; und
1 bis 40 Gew.-% einer Pulverkomponente.

20. Prozess nach Anspruch 19, umfassend den weiteren Schritt des Formens des resultierenden Gemischs in eine feste Form, die zur topischen Anwendung geeignet ist.

## Revendications

1. Composition cosmétique comprenant :
de 0,05% à 5% en rapport poids/poids d'une résine cationique hydrophile ;
de 30% à 85% en rapport poids/poids d'un composé huilé ;
de 1% à 40% en rapport poids/poids d'un composé de cire ; et
de 1% à 40% en rapport poids/poids d'un composé en poudre.

2. Composition cosmétique selon la revendication 1, dans laquelle la résine cationique hydrophile est soluble dans l'eau et/ou gonflable dans l'eau.

3. Composition cosmétique telle que revendiquée dans toute revendication précédente, dans laquelle la résine cationique hydrophile comporte un ou plusieurs polymères polyquaternium et/ou des sels polymères.

4. Composition cosmétique telle que revendiquée dans toute revendication précédente, dans laquelle la résine cationique hydrophile comporte un polymère quelconque d'ammonium quaternaire polymère approprié, un sel d'ammonium polymère quaternaire ou tout mélange de ceux-ci.

5. Composition cosmétique telle que revendiquée dans toute revendication précédente, dans laquelle la résine cationique hydrophile comporte un homopolymère, un copolymère ou tout mélange approprié de ceux-ci.

6. Composition cosmétique telle que revendiquée dans toute revendication précédente, dans laquelle la résine cationique hydrophile comporte une ou plusieurs des substances suivantes ou tout mélange de celles-ci :
Polyquaternium-1 ; polyquaternium-2 ; polyquaternium-4 ; polyquaternium-5 ; Polyquaternium-6; polyquaternium-7 ; polyquaternium-8 ; polyquaternium-9 ; Polyquaternium-10 ; polyquaternium-11 ; polyquaternium-12 ; polyquaternium-13 ;Polyquaternium-14 ;polyquaternium-15 ;polyquaternium-16 ; polyquaternium-17 ; polyquaternium-18 ; polyquaternium-19 ; polyquaternium-20 ; Polyquaternium-22 ; polyquaternium-24 ; polyquaternium-27 ; polyquaternium-28 ; Polyquaternium-29 ; polyquaternium-30 ; polyquaternium-31 ; polyquaternium-32 ;Polyquaternium-33 ; Polyquaternium-34 ; polyquaternium-35 ; polyquaternium-36 ; polyquaternium-37 ; et Polyquaternium-39.

7. Composition cosmétique telle que revendiquée dans toute revendication précédente dans laquelle la résine cationique hydrophile comporte un polyquaternium-37.

8. Composition cosmétique telle que revendiquée dans toute revendication précédente dans laquelle la résine cationique hydrophile comporte une matière de base sélectionnée parmi un ester émollient et/ou une huile émolliente.

9. Composition cosmétique telle que revendiquée dans toute revendication précédente, qui est solide à température ambiante et convient à un usage en tant que rouge à lèvres.

10. Composition cosmétique telle que revendiquée dans toute revendication précédente, dans laquelle le composé huilé représente de 50% à 80% en rapport poids/poids.

11. Composition cosmétique telle que revendiquée dans toute revendication précédente, dans laquelle le composé de cire représente de 5% à 40% en rapport poids/poids.

12. Composition cosmétique telle que revendiquée dans toute revendication précédente, dans laquelle le composé de cire représente de 10% à 30% en rapport poids/poids.

13. Composition cosmétique telle que revendiquée dans toute revendication précédente, dans laquelle le composé en poudre comporte une poudre non pigmentaire en combinaison avec une poudre pigmentaire.

14. Composition cosmétique telle que revendiquée dans toute revendication précédente, dans laquelle le composé en poudre représente de 2% à 30% en rapport poids/poids.

15. Composition cosmétique telle que revendiquée dans toute revendication précédente, dans laquelle le composé en poudre est traité en surface avec un ou plusieurs des ingrédients sélectionnés parmi du stéarate d'aluminium, des acides aminés, des huiles minérales, des phospholipides, des huiles siliconées et tout mélange de ces substances.

16. Composition cosmétique telle que revendiquée dans toute revendication précédente, qui comporte de plus un ou plusieurs ingrédient(s) sélectionnés parmi : des agents modifiants du lustre, des agents modifiants de la texture, des agents humidifiants, des agents lissants, des conditionneurs, des vitamines, des agents écran solaires, des conservateurs, des anti-oxydants, des parfums et tout mélange de ces substances.

17. Produit cosmétique contenant une composition cosmétique telle que revendiquée dans toute revendication précédente, en association avec tout conteneur ou applicateur approprié de celle-ci.

18. Procédé de traitement cosmétique comportant l'application topique d'un composition cosmétique telle que revendiquée dans l'une quelconque des revendications 1 à 16 ou usage d'un produit cosmétique tel que revendiqué en revendication 17.

19. Procédé de préparation d'une composition cosmétique telle que revendiquée dans l'une quelconque des revendications 1 à 17, comprenant le mélange intense des ingrédients suivants :
de 0,05% à 5% en rapport poids/poids d'une résine cationique soluble dans l'eau;
de 30% à 85% en rapport poids/poids d'un composé huilé ;
de 1% à 40% en rapport poids/poids d'un composé de cire ; et
de 1% à 40% en rapport poids/poids d'un composé en poudre.

20. Procédé tel que revendiqué en revendication 19, comprenant l'étape supplémentaire de moulage du mélange résultant dans une forme solide appropriée pour une application topique.
